(19)

Europäisches
Patentamt
European
Patent Office
Office européen
des brevets

(11)  **EP 2 330 910 B1**

(12)  **EUROPEAN PATENT SPECIFICATION**

(45) Date of publication and mention
of the grant of the patent:
**16.08.2017  Bulletin 2017/33**

(51) Int Cl.:
***A61Q 19/08*** (2006.01)

(21) Application number: **09812783.0**

(22) Date of filing: **13.09.2009**

(86) International application number:
**PCT/IL2009/000891**

(87) International publication number:
**WO 2010/029554 (18.03.2010 Gazette 2010/11)**

(54) **COSMETIC USE OF LEUCOJUM BULB EXTRACTS**

KOSMETISCHE VERWENDUNG VON MÄRZENBECHEREXTRAKTEN

UTILISATION COSMÉTIQUE DES EXTRAITS DU BULBE DE LEUCOJUM

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority:  **11.09.2008  US 95954 P**

(43) Date of publication of application:
**15.06.2011  Bulletin 2011/24**

(73) Proprietor: **Israeli Biotechnology Research Ltd.
Yavne 8122503 (IL)**

(72) Inventors:
• **SOUDANT, Etienne
F-75008 Paris (FR)**
• **FISHBEIN MANOR, Danit
83815 (IL)**
• **ALILUIKO, Alex
76659 Rehovot (IL)**
• **VON OPPEN-BEZALEL, Lea
14163 Berlin (DE)**
• **PERRY, Inon
65150 Tel-Aviv (IL)**

(74) Representative: **Becker Kurig Straus
Patentanwälte
Bavariastrasse 7
80336 München (DE)**

(56) References cited:
WO-A1-98/36761          BG-U1- 1 055
US-A1- 2006 045 896     US-A1- 2006 160 702
US-A1- 2006 160 702     US-A1- 2006 269 625
US-B1- 6 235 272        US-B2- 6 617 452

• **Anonymous: "Summer Snowflake Extract
Targets Wrinkles", Cosmetics & Toiletries -
Science Applied , 18 November 2008
(2008-11-18), XP002686256, Retrieved from the
Internet:
URL:http://www.cosmeticsandtoiletries.com/
formulating/ingredient/active/34704034.htm l
[retrieved on 2012-10-26]**
• **Anonymous: "For skin as smooth and bright as
snow", SpecialChem , 21 November 2008
(2008-11-21), XP002686257, Retrieved from the
Internet:
URL:http://www.specialchem4cosmetics.com/s
ervices/news.aspx?id=3607 [retrieved on
2012-10-26]**
• **Anonymous: "A new product from IBR For skin
as smooth and bright as snow.", IBR - News List ,
15 October 2008 (2008-10-15), XP002686258,
Retrieved from the Internet:
URL:http://www.ibrweb.com/newslist1.htm
[retrieved on 2012-10-26]**

EP 2 330 910 B1

## Description

### FIELD OF THE INVENTION

The present invention relates to a cosmetic method for slackening a cutaneous tissue comprising administering via subcutaneous, transdermal or intradermal delivery an aqueous extract of *Leucojum* bulbs effective in inhibiting contraction of muscle cells, and effective in ameliorating, reducing and/or eliminating wrinkles, fine lines and skin creases.

### BACKGROUND OF THE INVENTION

Skin is composed of the epidermis and the dermis. Below these layers lies the hypodermis, which is not usually classified as a layer of skin. The hypodermis is also commonly referred to as subcutaneous fat layer, or subcutaneous tissue. The outermost epidermis is made up of stratified squamous epithelium with an underlying basement membrane. It contains no blood vessels, and is nourished by diffusion from the dermis. The epidermis is mainly composed of keratinocytes, with melanocytes and langerhans cells also present. This layer of skin functions as a barrier between the body and the external environment, keeping water in the body and preventing penetration of harmful chemicals and pathogens.

The dermis lies below the epidermis and contains a number of structures including blood vessels, nerves, hair follicles, smooth muscle, glands and lymphatic tissue. The dermis (or corium) is typically 3-5 mm thick and is the major component of human skin. It is composed of a network of connective tissue, predominantly collagen fibrils providing support and elastic tissue providing flexibility. The main cell types composing the dermis are fibroblasts, adipocytes (fat storage) and macrophages. The hypodermis lies below the dermis. Its purpose is to attach the skin to underlying bone and muscle as well as supplying it with blood vessels and nerves. It is made up of loose connective tissue and elastin. The hypodermis is composed of fibroblasts, macrophages and adipocytes, the adipocytes playing a major role in the fat storage function of the hypodermis. The fat serves as a filling material and as insulation of the body from the external environment.

Facial aging occurs as the result of several factors, among them are inherent changes within the skin, effects of gravity, activity of facial muscles leading to the formation of dynamic lines, soft tissue loss or shift, bone loss, loss of tissue elasticity and exposure to harsh environmental conditions, particularly the sun or ultraviolet radiation and pollutants. The skin ages when the epidermis begins to thin, causing the junction with the dermis to flatten. Collagen decreases as a person ages and the bundles of collagen, which gives the skin turgor, become looser and lose strength. When the skin loses elasticity, it is less able to resist stretching. Coupled with gravity, muscle pull and tissue changes, the skin begin to wrinkle. Water loss and breakdown of bonds between cells also reduces the barrier function of the skin, which can cause the skin's pore size to increase.

Reactive oxygen species (ROS) are generated in increased amounts in the body cells as a result of diseases, aging, and external aggravations. The skin in particular is vulnerable to damage by reactive oxygen species, contributing to its non-desired aged-appearance. Anti-oxidants are therefore commonly used in skin care.

Botulinum toxin, (also known by the trade name, BOTOX® Allergan, Irvine, Calif.), is currently in vogue for treating wrinkles and fine lines, but was initially used to treat spasms. This toxin acts on states of muscular spasticity by specifically inhibiting neurotransmission in nerve cells, particularly inhibiting the release of acetylcholine, thereby causing contracted muscles to relax (e.g., U.S. Patent Nos. 6,395,277; 6,939,852; and 7,384,918). Botulinum toxin also can act on wrinkles of the glabella (wrinkles between the eyebrows) when injected subcutaneously. However, the full extent of adverse effects related to long-term use of botulinum toxin and products or treatments containing this material are still not known, and use under certain condition may even cause death. Botulinum toxin treatment has been associated with a number of side effects including, transient fatigue, dysphagia, neck weakness, hoarseness, and localized pain. In addition, many patients who preliminarily respond to botulinum toxin subsequently become non-responsive to treatment.

Use of spilanthol, in the form of an *Acmella oleracea* extract has been suggested as an inhibitor of contractions of subcutaneous muscles, notably those of the face, and thus proposed as an anti-wrinkle product having botulinum-like activity, while not being toxic (U.S. Patent Application Publication No. 2008/0069912).

*Leucojum* plants, also known as "Snowflake", are bulbous plants belonging to the Amaryllidaceae family. The only species currently classified in the genus *Leucojum* are *Leucojum vernum* (Spring Snowflake) and *Leucojum aestivum* (Summer Snowflake).

*L. aestivum* whole plant extracts are known to contain the active ingredient galanthamine, having various activities. Methods for galanthamine purification from extracts of various parts of the *Leucojum* plant, including bulbs, have been disclosed (for example, U.S. Patent Nos. 6,573,376 and 6,617,452; U.S. Patent Application Publication No. 2009/0216012.

U.S. Patent No. 6,117,428 discloses ruminant repellant composition for protection of foliage from browsing by ruminants, e.g. deer, the ruminant repellent composition containing compounds found in Amaryllidaceae family, including, inter alia, *L. aestivum.*

U.S. Patent No. 6,159,476 discloses improving muscle performance in athletes by administration of dietary supplement or herbal composition comprising galanthamine, particularly compositions comprising whole plant extract of *L. aestivum.*

U.S. Patent Number 7,029,708 discloses improving estrogen-deficiency related neurodegeneration and cognitive dysfunction in women by administering herbal extract compositions, particularly herbal extract of *L. aestivum*, in which the active ingredient of the herbal extract is galanthamine.

Galanthamine has been also used for the treatment of various diseases of the nervous system including Alzheimer's disease (U.S. Patent No. 5,958,903) and Parkinson's disease (U.S. Patent No. 5,965,571). This function of galanthamine was attributed to its activity as an inhibitor of acetylcholinesterase.

Some of the inventors of the present invention have previously disclosed (U.S. Patent Application Publication No. 2006/0160702) anti-proliferative agents derived from plants, wherein the agents are capable of inducing in a plant organ a state of dormancy or maintaining the organ in the state of dormancy. The invention further discloses compositions comprising the anti-proliferative agents and the use of said compositions to inhibit undesired or deleterious cell proliferation in plant or mammal tissue. Anti-proliferative extract from dormant *Leucojum* bulbs was shown among others.

U.S. Patent Application Publication No. 2008/0254055 indicates an extract of *Leucojum aestivum* as skin care active in a list of many others, without providing any description of activity and/or efficacy of this extract.

BG 1 055 U1 discloses a muscle strengthening effect of a *Leucojum aestivum* extract, as well as a cosmetic agent comprising said extract for topic administration onto the skin surface.

WO 98/36761 discloses an antiproliferative effect of aqueous extracts of *Leucojum aestivum*, which is used for treating age related phenomena.

Natural plant extracts effective in enhancing relaxation of muscles are highly desirable in the cosmetic and pharmaceutical industries. Particularly, there is a demand for non-toxic substances effective as relaxant of cutaneous and subcutaneous muscles for treating and preventing the visible signs of aging and weathered skin such as wrinkles and lines.

## SUMMARY OF THE INVENTION

The present invention provides a cosmetic method for slackening a cutaneous tissue comprising the step of administering via subcutaneous, intradermal or transdermal delivery a composition comprising an aqueous extract of *Leucojum aestivum* bulbs in an amount effective in inhibiting contraction of muscle cells.

The present invention is based in part on the unexpected discovery that aqueous extract of bulbs of the Amaryllidaceae plant *Leucojum,* particularly bulbs of *L. aestivum*, is effective in inhibition or blockage of muscular contractions. As described above, hitherto *Leucojum* extracts were known for the high content of galanthamine, an inhibitor of acetylcholinesterase. Nowhere is the background art is it disclosed or suggested that aqueous extracts of *Leucojum* bulbs is effective in inhibiting muscle contraction, and thus in treating and preventing wrinkles. Surprisingly, the muscle relaxant activity was obtained independently of the bulb dormancy stage.

The extract used in the context of the present invention is essentially non-cytotoxic. Thus, the extracts used in the context of the present invention can, *inter alia*, replace BOTOX® as, like BOTOX®, the extract shows muscular contraction inhibiting activity, while being devoid of the Botulinum toxicity.

Moreover, the present invention now discloses that this extract facilitates the expression of the enzyme manganese-superoxide dismutase (MnSOD), an anti-oxidant enzyme having a key-role in the scavenging of superoxide radicals. Thus, the extracts used in the context of the present invention are advantageous over previously known compounds having muscular contraction inhibiting activity as not only the extracts are non-cytotoxic, they show anti-oxidative activity, and thus may further protect the skin from aging.

Thus, according to one aspect, the present invention provides a cosmetic method according to claim 1.

Further embodiments of the present invention are disclosed in the depending claims.

Other objects, features and advantages of the present invention will become clear from the following description and drawings.

## BRIEF DESCRIPTION OF THE FIGURES

FIG. 1 shows the effect of 1.5% of *Leucojum aestivum* extract on muscle contraction in a muscle-nerve co-culture

FIG. 2 and FIG. 3 show 2-Dimension presentation of skin surface after treatment with product "T" (composition comprising 2% *Leucojum aestivum* extract) of subject No. 8 and 4 respectively. Fig. 2-3 A: Before treatment (Day 0). Fig. 2-3 B: After treatment (Day 28).

**FIG. 4** shows examples of 3-Dimensionl presentation of skin after treatment with product "T" (composition comprising 2% *Leucojum aestivum* extract) of subject No. 8. Fig. 4 A: Before treatment (Day 0). Fig. 4 B: After treatment (Day 28).

**FIG. 5** shows the overall effect of product "T" (composition comprising 2% *Leucojum aestivum* extract) on micro-relief furrows and medium depth wrinkles compared to control (placebo, composition only).

## DETAILED DESCRIPTION OF THE INVENTION

The present invention provides a cosmetic method for slackening a cutaneous tissue comprising administering via subcutaneous, intradermal or transdermal delivery a composition comprising an aqueous extract of *Leucojum aestivum* bulbs shown to be effective in inhibiting muscle contractions. The present invention now shows that the extracts are effective in the treatment, including preventing, reducing, ameliorating and/or eliminating signs of dermatological aging of the skin, including wrinkles, fine lines and other signs of dermatological aging (i.e., intrinsic aging) or sunlight exposure of the skin (i.e., extrinsic aging), by virtue of their inhibiting effect on muscle contractions and their anti-oxidative activity.

The extracts used in the context of the present invention modulate muscle contraction so as to relax the muscle fibers in cutaneous or subcutaneous muscle and/or nerve tissue, attenuating wrinkles, as well as fine lines, folds, furrows, and the like. By relaxing or preventing the contraction or hypercontraction of the cutaneous or subcutaneous muscle cells of areas such as the face, or hands, feet, etc., the *Leucojum* extracts and compositions containing same can effectively smooth out the landscape of the skin in those areas where muscle contraction is involved in the formation of wrinkles and the like. Thus, since muscles and muscle cell contractility is associated with the formation of wrinkles, fine lines, etc., the relaxation or decontraction of the contractility of cutaneous or subcutaneous muscle tissue by the compositions used in the context of the invention can serve to loosen or slacken the contracting muscle tissue and alleviate, reduce, ameliorate, inhibit, or eradicate the wrinkles and fine lines. The anti-oxidative properties of the extracts contribute further to the elimination of the visible dermatological effects of aging.

The *Leucojum aestivum* bulbs may be in a dormant or non-dormant state.

The *Leucojum aestivum* bulb extract used in the context of the present invention is characterized as inhibitor of muscle contractile activity. This effect is highly unexpected, as previously described whole-plant extracts of *L. aestivum* were shown to increase muscular quality, and thus to increase the effectiveness of muscle fiber contraction (e.g. U.S. Patent No. 6,159,476).

As used herein, the terms "topical administration" or "administered topically" refer to applying the *Leucojum* extract or compositions comprising same to a localized area of the body or to the surface of a body part. Thus, these terms encompass local administration to the surface of the skin as well as to local administration to the target muscle layer using targeted delivery system including, but not limited to, liposomes, microspheres, transdermal patches, micro- and nano- injections and needleless injection. Systemic routes of administration, such as oral and intravenous routes of administration, are excluded from the scope of the present invention.

Unexpectedly, no significant difference was found in the muscle contraction inhibitory activity between extracts obtained from dormant or non dormant bulbs of *Leucojum aestivum*, indicating that this activity is not related to the anti-proliferative activity of extracts obtained from dormant bulbs previously disclosed by some of the inventors of the present invention.

In addition, the raw *Leucojum* extract used in the context of the present invention was found to be essentially devoid of galanthamine.

The method of the present invention is for cosmetically treating skin. Skin in a variety of areas of the body can be effectively treated, including the neck, arms, legs, hands, feet, torso (chest), back, and the like.

The compositions and methods disclosed herein improve the aesthetic appearance of the skin by treating at least one of the following: signs of dermatological aging, especially chronological, actinic or hormonal aging, or signs of extrinsic aging, such as sun exposure. In particular, improvements to the aesthetic appearance of skin include at least one of the following: make facial lines appear less noticeable, make facial lines and/or wrinkles feel plumped, improve appearance of suborbital lines and/or periorbital lines, improve appearance of crow's feet, reduce and/or diminish the appearance of wrinkles, particularly facial wrinkles on the cheeks, forehead (e.g., perpendicular wrinkles between eyes, horizontal wrinkles above the eyes), and/or around the mouth, (e.g., marionette lines), and deep wrinkles, folds, or creases, reduce and/or eliminate fine and/or deep lines, folds and creases, and smooth skin, e.g., to the extent that wrinkling/lines are reduced.

The extracts as used in the context of the present invention are produced from the bulbs of *Leucojum.* Extraction can be performed from *Leucojum aestivum* bulbs, independently on the bulb dormant state. The bulbs are washed and peeled, and peeled bulbs are crashed in pure water until homogenized slurry is formed. The active ingredients are extracted into the aqueous solution while stirring the slurry and then coarse solids are removed from the slurry by decantation. Fine solids and proteins are removed by heating the suspension to a temperature of between 70-130°C and subsequent centrifugation. The obtained extract is diluted in water and/or glycerin according to the dry weight, and filtered.

The dry weight of the extract can be in the range of from about 7 mg/g to about 15 mg/g.

Contracted muscle cells or tissue is associated with formation of wrinkles, fine lines, etc. Relaxation or decontraction of contracted muscle, serves to smooth out the landscape, or microrelief, of the skin, thereby affecting the amelioration, reduction, and/or eradication of wrinkling and fine line formation caused by contracted muscle tissue in skin.

The compositions used in the context of the present invention may be formulated in any cosmetically and/or dermatologically suitable form as is known to a person skilled in the art. The formulation may be in the form of a lotion, gel or cream, in an ointment or oil base, as well as in a sprayable liquid form. Other suitable cosmetic product forms for the compositions used in the context of this invention include, for example, an emulsion, a mousse, a lip balm, a lip gloss, a lotion, a mask, an ointment, a pomade, a solution, a serum or a spray. The compositions can be incorporated into or mixed with a transdermal patch or other delivery systems such as micro and nano-injections as well as needleless injections.

In addition to the *Leucojum* bulb extract as active agent, as described herein, the compositions used in the context of the present invention can contain suitable pharmaceutically or cosmetically acceptable carriers, diluents, or excipients comprising auxiliaries which facilitate processing of the active compounds into preparations which can be used pharmaceutically or cosmetically. Further details on techniques for formulation and administration are provided in the latest edition of Remington's Pharmaceutical Sciences (Mack Publishing Co.; Easton, Pa.). The compositions containing the *Leucojum* bulb extract used in the context of the present invention can be manufactured in a manner that is known in the art, e.g., by means of conventional mixing, dissolving, granulating, dragee-making, levigating, emulsifying, encapsulating, entrapping, or lyophilizing processes.

The compositions as used in the context of the invention may further comprise one or more compatible cosmetically or pharmaceutically acceptable additives including, but not limited to fats, emulsifiers and co-emulsifiers, hydrophilic or lipophilic gelling agents, colorants, fragrances, emollients, humectants, preservatives, vitamins, chelators, thickeners, fillers, solvents, hydrophilic and lipophilic filters, dyestuffs, neutralizers, penetration enhancing agents polymers and the like, as well as other botanicals such as aloe, chamomile, and the like.

The fats can be selected from the group consisting of, but not limited to, mineral oils, oils of animal origin (lanolin), synthetic oils (isopropyl myristate, octyldodecyl, isostearyl isostearate, decyl oleate or isopropyl palmitate), silicone oils (cyclomethicone or dimethicone) and fluorinated oils. Fatty alcohol, fatty acids, waxes and gums, notably silicone gums and elastomers can be used as fats.

The emulsifiers and co-emulsifiers can be selected from the group consisting of, but not limited to, polyglycerol fatty acid esters, sucrose fatty acid esters, sorbitane fatty acid esters, oxyethylene sorbitan fatty acid esters, PEG fatty alcohol ethers, glycerol fatty acid esters, alkyl sulphates, alkyl ether sulphates, alkyl phosphates, alkyl polyglucosides and dimethicone copolyols.

The hydrophilic gelling agents can be selected from the group consisting of, but not limited to, carboxyvinyl polymers (carbomer), acrylic copolymers such as acrylate/alkylacrylate copolymers, polyacrylamids, polysaccharides such as xanthan gum, guar gum, natural gums such as cellulose gum and derivatives, clays and 2-acrylamido-2-methylpropane acid copolymers. The lipophilic gelling agents are selected from the group consisting of, but not limited to, modified clays such as bentones, fatty acid metal salts, hydrophobic silica and ethylcellulose. The fillers can be selected from the group consisting of, but not limited to, talc, kaolin, mica, serecite, magnesium carbonate, aluminum silicate and organic powders such as nylon. The dyestuffs can be selected from the group consisting of, but not limited to, lipophilic dyes, hydrophilic dyes, pigments and mother-of-pearl commonly used in cosmetic or dermatological compositions, and their mixtures. The neutralizers are selected from the group consisting of, but not limited to, soda, triethanolamine, aminomethyl propanol and potassium hydroxide. The penetration enhancing agents can be selected from the group consisting of, but not limited to, alcohols and glycols (ethanol and propylene glycol), ethoxydiglycol, alcohols and fatty acids (oleic acid), fatty acid esters and dimethyl isosorbide.

Preservatives compatible with cosmetic and dermatological compositions are well known in the art. Respective preservatives can be selected from the group consisting of benzoic acid, its salts and esters; sorbic acid and its salts; parabens and their salts; triclosan; imidazolidinyl urea; phenoxyethanol; DMDM hydantoin; diazolidinyl urea and chlorphenesin.

The filters can be conventionally used UVA and UVB filters selected from the group consisting of, but not limited to, organic filters: benzophenone-3, butyl methoxydibenzoyl methane, octocrylene, octyl methoxycinnamate, 4-methylbenzylidene camphor, octyl salicylate, terephthalylidene dicamphor sulfonic acid and drometrizole trisiloxane; non-organic filters: titanium oxide and zinc oxide.

The solvents can be selected from the group consisting of, but not limited to, water, ethanol, glycerin, propylene glycol, butylene glycol and sorbitol. According to typical; embodiments, the solvents are water and/or glycerin.

The compositions as used in the context of the present invention can also be formulated into liposomes, which can comprise other additives or substances, and/or which can be modified to more specifically reach or remain at a site following administration. Liposomes and delivery systems and vehicles involving liposomes are well-known in the art. In brief, liposomes are unilamellar or multilamellar lipid vesicles which entrap a significant fraction of aqueous solution. The vesicular microreservoirs of liposomes can contain a variety of water-soluble materials, which are suspended within the emulsion (e.g., reviewed in G. Gregorius (Ed.), 1991, Liposome Technology, Vols. I, II, III, CRC Press, Boca Raton, Fla.; Davis S. S. and Walker I. M., 1987, Methods in Enzymology, 149:51-64; Mayhew E. et al., 1987, Methods in Enzymology, 149:64-77; and Shafer-Korting M. et al., 1989, J. Am. Acad. Dermatol., 21:1271-1275). The preparation of liposomes and the variety of uses of liposomes in biological systems have been described (e.g., in U.S. Patent Numbers 4,708,861, 4,224,179, and 4,235,871). Accordingly, such liposomes can be formulated into any of the dermatological or cosmetic compositions as described herein. Additionally or alternatively, the compositions used in the context of the present invention can be encapsulated, particularly by micro- and nano- encapsulation.

*Leucojum* bulb extract-containing compositions can be formulated and delivered to the skin and muscles using various delivery systems available such as micro- and nano-injections, needleless injections transdermal delivery and channeling systems helping bringing the active ingredients to their point of action.

Alternatively, the *Leucojum* bulb extract-containing compositions can be injected subcutaneously (s.c.) or intradermally (i.d.) at a site in need, i.e. a skin site having wrinkle, fine line, etc.

One skilled in the art is capable of determining the effective dose or amount of the compositions to be used with the methods of the present invention as well as the application regime. An effective dose refers to that amount of active ingredient, i.e. aqueous *Leucojum* bulb extract identified in accordance with the present invention, which, for instance, treats, prevents, ameliorates, reduces, or eliminates wrinkles, fine lines, creases, and the like. The exact dose will be determined by the practitioner according to certain factors related to the individual requiring treatment, including the severity of the individual's particular need, general health of the individual, age, weight, and gender of the individual, diet, time and frequency of administration, drug combination(s), reaction sensitivities, and tolerance/response to treatment. As a general guide, long-acting cosmetic compositions can be administered once daily, every 2 to 4 days, every week, or once every two weeks, depending on half-life and clearance rate of the particular formulation. Variations in these dosage levels can be adjusted using standard empirical routines for optimization, as is well known to a person skilled in the art. Guidance as to particular dosages and methods of delivery is provided in the literature and is generally available to practitioners in the art. By way of example, the *Leucojum* bulb extract is present in the composition in an amount of from about 0.0001 wt % to about 0.3 wt % when the extract is in its dry form based on the total weight of the composition, and from about 0.01 wt % to about 30 wt % when the extract is in its liquid form based on the total weight of the composition. The composition can be applied once a day every day, twice a day, or during a period of from about 7 days to about 30 days.

Advantageously, the *Leucojum* extract as used in the context of the present invention has now been shown to have anti-oxidative activity, due to its effect on MnSOD expression as described herein below. Nevertheless, the compositions used in the context of the present invention may further comprise additional active-ingredients, including additional anti-oxidants. Additional active ingredients include, but are not limited to, depigmenting agents, moisturizers, anti-seborrheic agents, anti-acne agents, keratolytic and/or desquamating agents, anti-irritant agents, soothing agents, and additional anti-ageing active ingredients such as retinol and other anti-wrinkle agents.

According to certain embodiments, the aqueous extract of non-dormant *Leucojum* bulbs can be in a liquid form. Alternatively, the extract can be in a dried powder form.

According to certain embodiments, the cosmetic composition used in the context of the present invention comprises from about 0.01% to about 99.9% (w/w), typically from about 0.01% to about 30% (w/w), more typically from about 0.1% to about 10% (w/w) of the extract when the extract is in a liquid form. According to other embodiments, the composition used in the context of the present invention comprises from about 0.0001% to about 0.99% (w/w), typically from about 0.0001% to about 0.3% (w/w), more typically from about 0.01% to about 0.1% (w/w) of the extract when said extract in a dry powder form.

The following examples are presented in order to more fully illustrate some embodiments of the invention.

# EXAMPLES

## Example 1: Production of *Leucojum aestivum* bulb extract

*Leucojum aestivum* bulbs were washed and peeled. Peeled bulbs were then crashed in pure water until homogenized slurry is formed. Extraction was performed with spiral stirring for 15-60 minutes at a temperature range of 15-45°C. Removal of coarse solids was performed by decantation. Fine solids removal from the slurry was performed as follows:

- Heating the slurry in a temperature ranging between 70-130°C;
- Decanting the liquid phase extract to a new clean container;
- Centrifuging the obtained liquid by discs centrifuge with flow rate ranging between 3-10Kg/minute.

Extract was diluted in water or/and glycerin to obtain a dry weight of from 7.0 mg/g to 15.0 mg/g. Final solution was filtrated through 1, 0.45 and 0.2 $\mu$m filters placed one after the other (Suprolife filters, Pall).

The final extract has a pH of from about 4.50 to about 6.50. The color of the extract is pale yellow to yellow brown, and it has non limited solubility in water. No galanthamine was detected in the extract, with a detection threshold value below 4 p.p.m.

## Example 2: Assessment of the effect of *L. aestivum* bulb extract on muscle contraction

*Leucojum aestivum* bulb extract, obtained as described hereinabove was used in this study. The objective was to evaluate the effect of this extract on muscular contraction frequency.

The nerve-muscle co-culture model is a model suited to studying the influence of a substance on a muscle contraction frequency. The number of muscular contractions is counted during a given time, before and after applying the examined substance to the co-culture. The number of contractions in the presence of the substance is expressed in percentage compared to the initial number of contractions. An inhibitory effect on muscle contraction is determined if a significant reduction or blocking of the muscular contraction frequency is observed in at least 2 wells out of 3 wells containing the nerve-muscle co-culture.

Materials and methods

Preparation of the cellular cultures

Human muscular cells were seeded in 24-wells culture plates. Cells were incubated at 37°C, 5% $CO_2$ during the entire culture duration.

After 5 days of culture, spinal cords were isolated from rat embryos, and were cut in small parts (explants). One explant per culture well was placed on the muscular cells, and both cell types were cultivated in 24-wells plate for 21 days.

During the cultivation period, the muscular cells merge and form muscular fibers. The motor neurons present in the spinal cord emit nervous extensions which contact the muscular fibers. Neuro-muscular junctions are formed and muscular contractions appear.

After 21 days of co-culture, the model was mature and functional. The muscular fibers innervated spontaneously and contracted in a regular way. These contractions were observed with an inverted microscope, and counted.

Preparation of the test extract

A stock solution of the test extract was prepared in the culture medium. This solution was applied to the nerve-muscle culture.

*Leucojum aestivum* extract was tested at 1.5%.

The culture medium alone served as a negative control.

The positive control was carisoprodol, a drug which blocks the muscular contraction in a reversible way. A stock solution of carisoprodol was prepared in DMSO at 1M. This solution was diluted at 1/200 into the culture medium (final concentration of $5x10^{-3}$M). Three culture wells were used per treatment (control/test extract).

Course of the trial

The trial started at the 21st day of the culture.

The cells were observed with a microscope in each well. The microscope is equipped with a motorized stage piloted by a LUCIA software. It is also combined with a camera and connected to a computer allowing the recording of video sequences.

For each selected culture well, a muscle fiber showing regular contractions is referenced. The XY data of this fiber were registered using the LUCIA software. The frequency of the contractions of this fiber was manually counted during 30 seconds, and a video sequence was recorded ($T_0$). The same operation was carried out for all wells.

The test extract and the references were applied to the nerve-muscle culture, and the trial system was incubated at 37°C, 5% $CO_2$.

After 2 hours, 6 hours and 24 hours of incubation, the contraction frequency was counted again during 30 seconds and a video sequence was also recorded for each counting.

Interpretation of the results

For each culture well, the number of muscular contractions counted in the presence of the examined extract is expressed in percentage compared to the number of contractions counted before extract application ($T_0$).

When the frequency of contractions is in the range of between 80%-120% compared to To, the modulation of the muscular contractions frequency is not considered as significant. This range is based on the fact that during the time, the muscular contraction frequency can vary slightly, independent on the conditions applied.

Frequency measures below 80% and over 120% are considered as significantly different from To.

The results are expressed as follows:

• No significant variation in the muscular contractions frequency: contractions percentage between 80 and 120%, symbolized by (=)

• A significant reduction in the muscular contractions frequency: contractions percentage < 80%, symbolized by (-).

• Blocking of the muscular contractions frequencies: contractions percentage = 0%, symbolized by (0)

• A significant increase in the muscular contractions frequencies: contractions percentage > 120%, symbolized by (+).

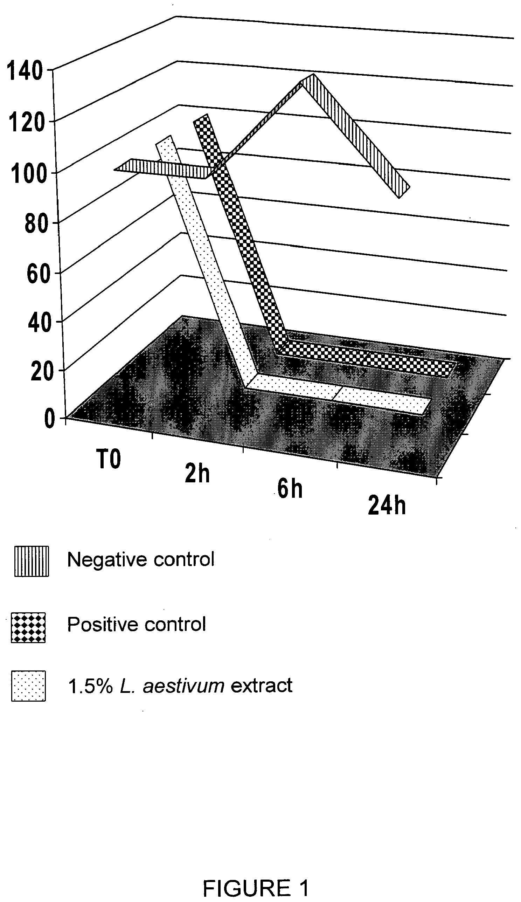

The product effect is assessed only if the same result is obtained in at least 2 out of 3 culture wells (i.e. 2 out of 3 muscle fibers). Tables 1-2 below and Figure 1 summarize the results obtained.

Results

Table 1: Control

| Condition | Incubation time | Contraction (%) | Result | conclusion |
|---|---|---|---|---|
| **Negative control-Culture medium** | $T_0$ | 100 | | |
| | | 100 | | |
| | | 100 | | |
| | 2h | 116 | = | **No variation** |
| | | 96 | = | |
| | | 93 | = | |
| | 6h | 241 | + | **Increase in contraction compared to $T_0$** |
| | | 137 | + | |
| | | 47 | - | |
| | 24h | 164 | + | **Reduction in contraction compared to $T_0$** |
| | | 63 | - | |
| | | 78 | - | |
| **Positive control: Carisoprodol** | $T_0$ | 100 | | |
| | | 100 | | |
| | | 100 | | |
| | 2h | 0 | 0 | **Blocked** |
| | | 0 | 0 | |

(continued)

| Condition | Incubation time | Contraction (%) | Result | conclusion |
|---|---|---|---|---|
| | | 0 | **0** | |
| | **6h** | 0 | **0** | **Blocked** |
| | | 0 | **0** | |
| | | 0 | **0** | |
| | **24h** | 0 | **0** | **Blocked** |
| | | 0 | **0** | |
| | | 0 | **0** | |

When the culture medium alone was applied to the test well (negative control), no significant variation in the frequency of muscle contractions was observed 2 hours after application. Some variation was observed after 6 hours and 24 hours of incubation.

The carisoprodol (positive control) produces a complete blockage of the muscle contractions after 2 hours, 6 hours and 24 hours of incubation. After rinsing off, a recovery of the muscle contractions was observed, indicating that the effect of carisoprodol is reversible.

Although some contraction frequency variations was observed with the negative control at 6 and 24 hours, the experimental procedure was validated as the examined product provoked a complete blockage of the contractions (see below), and not only a decrease.

Table 2: *Leucojum aestivum* extract

| Condition | Incubation time | Contraction (%) | Result | conclusion |
|---|---|---|---|---|
| ***Leucojum aestivum* extract 1.5 %** | $T_0$ | 100 | | |
| | | 100 | | |
| | | 100 | | |
| | **2h** | 0 | 0 | **blocked** |
| | | 0 | 0 | |
| | | 0 | 0 | |
| | **6h** | 0 | 0 | **blocked** |
| | | 0 | 0 | |
| | | 0 | 0 | |
| | **24h** | 0 | 0 | **blocked** |
| | | 0 | 0 | |
| | | 0 | 0 | |

Conclusions

In the experimental conditions described above *Leucojum aestivum* extract at 1.5%, blocked the muscular contractions frequency 2, 6 and 24 hours after application.

After rinsing off (incubation time 25 hours), the muscular contractions were blocked, indicating that the effect of the *Leucojum* extract at 1.5% is not reversible.

**Example 3: Evaluation of *L. aestivum* bulb extract on facial wrinkles**

*Leucojum aestivum* bulb extract, obtained as described hereinabove was used in this study at a concentration of 2% within a composition comprising the following ingredients: potassium palmitoyl; hydrolyzed wheat protein; glyceryl stearate; cetearyl alcohol; isohexadecane; caprylic/capric triglyceride; PPG-15 stearyl ether; propylene glycol; xanthan gum; magnesium aluminum silicate; dehydroacetic acid; benzoic acid; sorbic acid; benzyl alcohol; *Leucojum aestivum* bulb Extract 2%. Same composition without the *Leucojum aestivum* bulb Extract served as control.

The objective of this study was to evaluate and illustrate, on human subjects, the anti-wrinkle effect of a composition comprising the extract compared to a control, after 28 days of use. The study was a double blind and intra-individual study, i.e. each subject was her own control.

**Principle of the study**

Wrinkle measurement

Polymer silicone skin's prints were taken from the studied skin zones, before and after application of the products (test and control). The Skin's prints were then studied using the Skin Image Analyser® (S.I.A).

Briefly, oblique lighting (35°) creates shadows on the replica fore, and the shadows are photographed with a digital camera linked to a computer. This produces a digitized image in which the shades of gray are analyzed for several parameters characterizing the skin surface relief. 1 cm$^2$ area of each print was studied.

Using QUANTIRIDES® - MONADERM software, the studied parameters were number of cutaneous microrelief furrows and number of median wrinkles. Wrinkle depth was also observed.

Microrelief furrows have a depth inferior to 55 $\mu$m. Median wrinkle furrows have a depth between 55 and 110 $\mu$m. All furrows with a minimum surface of 0.03 mm$^2$ were detected.

3D Illustrations

Polymer silicone skin's prints (Silflo®) obtained as described above, of subjects which presented the best visual effect were studied using Primos® 3D Compact (Phaseshift Rapid In vivo Measurement of Skin, GFMesstechnik GmbH, Germany).

This technique calculates a phase image from images with interference fringe projection, allowing determination of the height of each point. The software allows obtaining 2D and 3D measurements. An automatic system of repositioning allows the precise re-identification of the zone of measurement. The system was as follows:

| | |
| --- | --- |
| **Projection system** | Digital Micromirror Devices (DMD®) of Texas Instruments Inc./USA with 800x600 micromirror |
| **Camera** | 640 x 480 pixels |
| **Measuring field** | 18 x13 mm$^2$ |
| **Acquisition time of measurement** | 17 ms, for one image 68 ms, for a complete 3D profile |
| **Vertical resolution** | 1 $\mu$m |
| **Lateral resolution** | 17 $\mu$m x 17 $\mu$m |
| **Use of polarized optics** | yes |

**Subject selection**

The minimal number of subjects for the study is 20. Inclusion criteria were as follows: healthy subject, that have given her informed, written consent; cooperative subject, aware of the necessity and duration of controls so that perfect adhesion to the protocol established by the clinical trial center could have been expected; female; age of 18 years old and above; having moderate crow's feet wrinkles.

Exclusion criteria were as follows: pregnant or nursing woman or woman planning to get pregnant during the study; cutaneous pathology on the studied zone (eczema, etc); subject using, on the measured zone, any product acting on the cutaneous relief (anti-wrinkle cream) or having stopped using same less than 1 month before the beginning of the study; subject having done filler injection and/or palpebral lifting; subject having changed, started or stopped her oral contraception or any hormonal treatment for less than 1.5 month before the beginning of the study; subject using topical or systemic treatment during the previous two weeks liable to interfere with the assessment of the cutaneous acceptability of the studied product; subject considered by the investigator to be non-compliant to the protocol; and subject experiencing excessive exposure to sunlight or UV-rays within the month previous to the study.

The usual cleansing product(s) were authorized for use on the face during the study (except for the visits at the laboratory). Use of dermopharmaceutical or cosmetic products other than the studied products was not authorized for application to the face during the study. Excessive exposure to sunlight or UV-rays during the study was also not authorized.

**Trial schedule**

Day 0 $D_0$, the day before the study starts)

- Subjects came to the laboratory without having applied any product to the face since the previous evening.

- They read, signed and dated the information sheet (instructions on the product use and restrictions related to the study) and informed consent forms in duplicate. These documents were also signed and dated by the person who conducted the informed consent discussion. The subjects received a copy.

- The technician in charge of the study verified the inclusion and non-inclusion criteria.

- Two zones on the face were defined: one zone treated with the test product and another treated with a control (Products "T" and "K" respectively described hereinbelow).

- Skin prints of the two studied zones were taken.

- The study products were given to the subjects with instructions to apply the products twice-daily on each defined hemi-face.

Day 28 ($D_{28}$)

- The subjects returned to the laboratory; the last application of products was done the evening before.

- Skin prints of the two studied zones were taken.

Since the subjects applied the test product at home, no compliance control could be carried out during the study.

**Study products**

The products were encoded as 1: product "T": the composition comprising 2% of *Leucojum aestivum* bulb extract described above; and 2: product "K", the composition only. Both compounds were white emulsions.

Before the beginning of the study, products were kept at room temperature in a dedicated air-conditioned room. This room was locked and access controlled.

The products were applied twice a day (morning and evening) during 28 days, one product to each hemi-face. The emulsions were applied under normal conditions of use. The application zones were randomized as described in Table 3 hereinbelow.

Table 3: Randomization of face application zone

| Subject | Product | |
|---|---|---|
| | Left | Right |
| 1 | T | K |
| 2 | K | T |
| 3 | T | K |
| 4 | K | T |
| 5 | T | K |
| 6 | K | T |

(continued)

| Subject | Product | |
|---|---|---|
| | Left | Right |
| 7 | T | K |
| 8 | K | T |
| 9 | T | K |
| 10 | K | T |
| 11 | T | K |
| 12 | K | T |
| 13 | T | K |
| 14 | K | T |
| 15 | T | K |
| 16 | K | T |
| 17 | T | K |
| 18 | K | T |
| 19 | T | K |
| 20 | K | T |
| 21 | T | K |
| 22 | K | T |
| Total K | 11 | 11 |
| Total T | 11 | 11 |

Twenty two (22) subjects started the study. One subject could not be reached on D28, and thus second measurements were taken for 21 subjects. The 21 subject complied with the study protocol. The mean age of the participants was 60 years old, with a minimum of 42 and maximum of 64 years old.

**Data analyses**

Calculation formulas

The variations ($\Delta$) in arbitrary units and in percentage on the mean ($\Delta\%$) of the different studied parameters were calculated according to the following formulas:

| Comparison Product 1/ product 2 | Comparison Before/ After |
|---|---|
| $\Delta = (ZPdt\text{-}1_{ti} - ZPdt\text{-}1_{t0}) - (ZPdt\text{-}2_{ti} - ZPdt\text{-}2_{t0})$ | $\Delta = (ZPdt\ ti - ZPdt_{t0})$ |
| $\Delta\% = \dfrac{(ZPdt\text{-}1_{ti} - ZPdt\text{-}1_{t0}) - (ZPdt\text{-}2_{ti} - ZPdt\text{-}2_{t0})}{ZPdt\text{-}1_{t0} + (ZPdt\text{-}2_{ti} - ZPdt\text{-}2_{t0})} \times 100$ | $\Delta\% = \dfrac{(ZPdt_{ti} - ZPdt_{t0})}{ZPdt_{t0}} \times 100$ |

With:

ZPdt-1:   value obtained on the zone treated by Product 1 (T);
ZPdt-2:   value obtained on the zone treated by Product 2 (K);
t0:   before product application;
ti:   at each measurement time after application.

Remarks:

The percentage of the variation ($\Delta\%$) is expressed in percentage of the variation of the zone treated by product 1 ($ZPdt\text{-}1_{ti}$ - $ZPdt\text{-}1_{t0}$) compared to the variation of the zone treated by product 2 ($ZPdt\text{-}2_{ti}$ - $ZPdt\text{-}2_{t0}$) or compared to the status before treatment:

$$(ZPdt\text{-}1_{ti} - ZPdt\text{-}1_{t0}) - (ZPdt\text{-}2_{ti} - ZPdt\text{-}2_{t0}) \quad \text{or} \quad (ZPdt_{ti} - ZPdt_{t0})$$

These variations are balanced at the initial value $ZPdt_{t0}$ (before treatment), corrected by the possible leeway between t0 and ti, independent of the treatment. This leeway is evaluated on the zone treated by product 2 ($ZPdt\text{-}2_i$ - $ZPdt\text{-}2_{t0}$), or on the treated zone at t0, which gives the denominator:

$$Pdt\text{-}1Z_{t0} + (ZPdt\text{-}2_{ti} - ZPdt\text{-}2_{t0}) \quad \text{or} \quad ZPdt_{t0}$$

This expression ($\Delta\%$), therefore, gives the variation, in percentage, of each zone treated with product 1 (T) compared to the initial conditions, taking into account the fluctuations (independent of treatment) observed for the zone treated by product 2 (K).

For each studied parameters, the values obtained from the zone treated with product T (Tables 5-6) and from the zone treated with product K (Tables 7-8) before application (day 0) and after the completion of the treatment (day 28) are presented.

These tables also show the descriptive statistics: means, medians, minima, maxima, standard errors of the means (SEM) and confidence intervals of 95% (CI 95%) of these values.

## Statistical method(s)

The statistical analysis determined the significance of the variation obtained from skin area treated with each of the study products.

Data were analyzed with a paired t-test. This method tests whether the mean of a sample difference between pairs of data is significantly different from the hypothetical mean, zero under the null hypothesis (H0).

The alternative hypothesis (H1) was that the average difference was either greater or less than 0 (two-tailed test). Before carrying out a test, a type I error of 5% is chosen (which corresponds to the risk of rejecting a true null hypothesis).

If $p \leq 0.05$, H0 was rejected. There was a significant difference between before and after the treatment or between the tested products.

If $p \geq 0.05$, H0 was accepted, the mean was not different from 0. Data did not show a significant difference between before and after the treatment or between the tested products. The software used was EXCEL 9.0 version 2003.

## Results

Anti-wrinkle effect

The studied parameters were:

- Number of micro-relief furrows.

- Number of medium wrinkles.

Table 4 presents number of micro-relief furrows and table 5 presents number of medium wrinkles measured on polymer silicone skin's prints of individual subjects from skin areas treated with product "T" (the composition comprising 2% *Leucojum aestivum* extract), before and after the treatment.

Table 4: Micro-relief Furrows of skin areas treated with product "T" .

| Subjects | Number of Micro-relief furrows | | |
| --- | --- | --- | --- |
| | D0 | D28 | D28-D0 |
| 1 | (17)* | UN | UN |
| 2 | 16 | 15 | -1 |
| 3 | 13 | 8 | -5 |
| 4 | 12 | 9 | -3 |
| 5 | 44 | 43 | -1 |
| 6 | 11 | 25 | 14 |
| 7 | 18 | 8 | -10 |
| 8 | 20 | 7 | -13 |
| 9 | 36 | 35 | -1 |
| 10 | 23 | 16 | -7 |
| 11 | 23 | 7 | -16 |
| 12 | 8 | 9 | 1 |
| 13 | 60 | 28 | -32 |
| 14 | 16 | 12 | -4 |
| 15 | 12 | 7 | -5 |
| 16 | 31 | 8 | -23 |
| 17 | 36 | 21 | -15 |
| 18 | 34 | 24 | -10 |
| 19 | 24 | 12 | -12 |
| 20 | 32 | 34 | 2 |
| 21 | 17 | 8 | -9 |
| 22 | 35 | 24 | -11 |
| **Mean** | **25** | **17** | **-8** |
| Median | 23 | 12 | -7 |
| Minimum | 8 | 7 | -32 |
| Maximum | 60 | 43 | 14 |
| **SEM** | **3** | **2** | **2** |
| 95% CI | 6 | 5 | 4 |
| | △D28 | | **-31%** |
| p= | | | 0.002 |
| **Percentage of subjects with a smoothing effect** | | | **86%** |

Table 5: Measurements of medium wrinkle of skin areas treated with product "T"

| Subjects | Number of Medium Wrinkles | | |
| --- | --- | --- | --- |
| | D0 | D28 | D28-D0 |
| 1 | (43)* | UN | UN |
| 2 | 31 | 34 | 3 |

(continued)

| Subjects | Number of Medium Wrinkles | | |
|---|---|---|---|
| | D0 | D28 | D28-D0 |
| 3 | 27 | 20 | -7 |
| 4 | 46 | 14 | -32 |
| 5 | 28 | 40 | 12 |
| 6 | 33 | 30 | -3 |
| 7 | 22 | 29 | 7 |
| 8 | 46 | 29 | -17 |
| 9 | 18 | 8 | -10 |
| 10 | 29 | 22 | -7 |
| 11 | 45 | 37 | -8 |
| 12 | 35 | 49 | 14 |
| 13 | 35 | 24 | -11 |
| 14 | 31 | 15 | -16 |
| 15 | 55 | 30 | -25 |
| 16 | 18 | 19 | 1 |
| 17 | 61 | 47 | -14 |
| 18 | 29 | 35 | 6 |
| 19 | 35 | 27 | -8 |
| 20 | 32 | 38 | 6 |
| 21 | 26 | 35 | 9 |
| 22 | 49 | 44 | -5 |
| **Mean** | **35** | **30** | **-5** |
| Median | 32 | 30 | -7 |
| Minimum | 18 | 8 | -32 |
| Maximum | 61 | 49 | 14 |
| **SEM** | **3** | **2** | **3** |
| 95% CI | 5 | 5 | 5 |
| **△D28** | | | **-14%** |
| **p=** | | | **0.071** |
| **Percentage of subjects with an anti-wrinkle effect** | | | **62%** |

Tables 6-7 present the same parameters measured on polymer silicone skin's prints of individual subjects from skin areas treated with product "K" (the control composition), before and after the treatment.

Table 6: Furrows microrelief of skin areas treated with product "K"

| Subjects | Number of Microrelief furrows | | |
|---|---|---|---|
| | D0 | D28 | D28-D0 |
| 1 | (23)* | UN | UN |
| 2 | 48 | 36 | -12 |

(continued)

| Subjects | Number of Microrelief furrows | | |
|---|---|---|---|
| | D0 | D28 | D28-D0 |
| 3 | 10 | 5 | -5 |
| 4 | 28 | 15 | -13 |
| 5 | 17 | 18 | 1 |
| 6 | 16 | 9 | -7 |
| 7 | 16 | 5 | -11 |
| 8 | 31 | 18 | -13 |
| 9 | 29 | 42 | 13 |
| 10 | 18 | 15 | -3 |
| 11 | 32 | 32 | 0 |
| 12 | 23 | 15 | -8 |
| 13 | 39 | 34 | -5 |
| 14 | 24 | 22 | -2 |
| 15 | 9 | 7 | -2 |
| 16 | 11 | 5 | -6 |
| 17 | 13 | 9 | -4 |
| 18 | 30 | 46 | 16 |
| 19 | 8 | 10 | 2 |
| 20 | 25 | 40 | 15 |
| 21 | 8 | 40 | 32 |
| 22 | 17 | 5 | -12 |
| **Mean** | **22** | **20** | **-1** |
| Median | 18 | 15 | -4 |
| Minimum | 8 | 5 | -13 |
| Maximum | 48 | 46 | 32 |
| **SEM** | **2** | **3** | **3** |
| 95% CI | 5 | 6 | 5 |
| △D28 | | | -5% |
| p= | | | 0.653 |
| **Percentage of subjects with a smoothing effect** | | | **67%** |

Table 7: Measurements of medium wrinkle of skin areas treated with product "K"

| Subjects | Number of Medium Wrinkles | | |
|---|---|---|---|
| | D0 | D28 | D28-D0 |
| 1 | (26)* | UN | UN |
| 2 | 44 | 60 | 16 |
| 3 | 27 | 21 | -6 |

(continued)

| Subjects | Number of Medium Wrinkles | | |
| --- | --- | --- | --- |
| | D0 | D28 | D28-D0 |
| 4 | 52 | 64 | 12 |
| 5 | 24 | 9 | -15 |
| 6 | 48 | 47 | -1 |
| 7 | 30 | 33 | 3 |
| 8 | 64 | 45 | -19 |
| 9 | 34 | 21 | -13 |
| 10 | 35 | 58 | 23 |
| 11 | 25 | 25 | 0 |
| 12 | 86 | 75 | -11 |
| 13 | 36 | 27 | -9 |
| 14 | 25 | 30 | 5 |
| 15 | 43 | 26 | -17 |
| 16 | 21 | 21 | 0 |
| 17 | 44 | 36 | -8 |
| 18 | 50 | 53 | 3 |
| 19 | 16 | 6 | -10 |
| 20 | 63 | 56 | -7 |
| 21 | 7 | 17 | 10 |
| 22 | 43 | 40 | -3 |
| **Mean** | **39** | **37** | **-2** |
| Median | 36 | 33 | -3 |
| Minimum | 7 | 6 | -19 |
| Maximum | 86 | 75 | 23 |
| **SEM** | **4** | **4** | **2** |
| 95% CI | 8 | 9 | 5 |
| △D28 | | | **-6%** |
| p= | | | **0.367** |
| **Percentage of subjects with an anti-wrinkle effect** | | | **57%** |

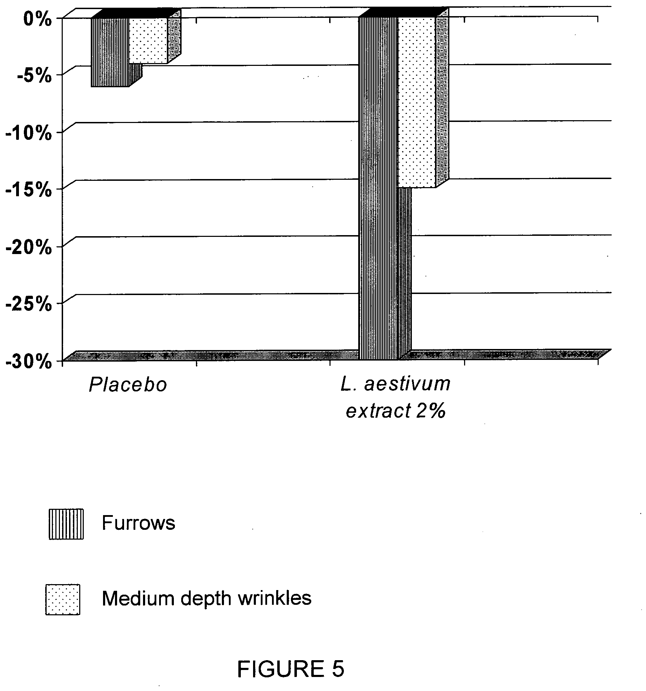

Table 8 below and Figure 5 summarize the mean variation of cutaneous relief parameters obtained from all subjects after 28 days of product application.

Table 8: Variations of cutaneous relief parameters after 28 days of use of each studied product, in comparison with the initial state

| | | | | Student *t*-test | | |
| | Product | Δ D28 (mean ± SEM) | Δ% on mean | P | Significant | % of subjects with the expected effect |
|---|---|---|---|---|---|---|
| Number of micro-relief furrows (μm) | T | -8 ± 2 | -31% | 0.002 | Yes | 86% |
| | K | -1 ± 3 | -5% | 0.653 | No | 67% |
| | T-K | -7 ± 3 | -28% | 0.052 | Yes | |
| Number of medium wrinkles | T | -5 ± 3 | -14% | 0.071 | tendency | 62% |
| | K | -2 ± 2 | -6% | 0.367 | No | 57% |
| | T-K | -3 ± 3 | -8% | 0.434 | No | |

Conclusions:

After 28 days of twice-daily use:

• Product "T", the composition comprising 2% of *Leucojum aestivum* extract induced a significant decrease in the number of micro-relief furrows (30% on average). A decrease was observed in 86% of the subjects. Moreover, a limit significant decrease in the number of medium wrinkles was also measured (-14% on average). A decrease in this parameter was observed in 62% of the subjects.

All these variations characterize a smoothing and a tendency to an anti-wrinkle effect of the composition comprising the *Leucojum aestivum* extract used in the context of the present invention (product "T"). The control composition (product "K") did not modify the cutaneous relief parameters.

The comparison between product "T" and product "K" highlighted a significant difference in the number of micro-relief furrows. Product "T" showed a better efficacy than product "K" in this parameter.

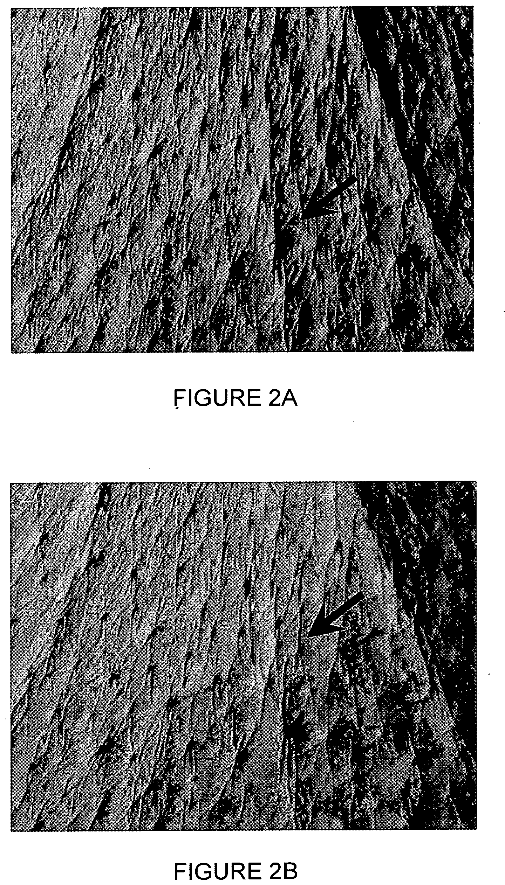

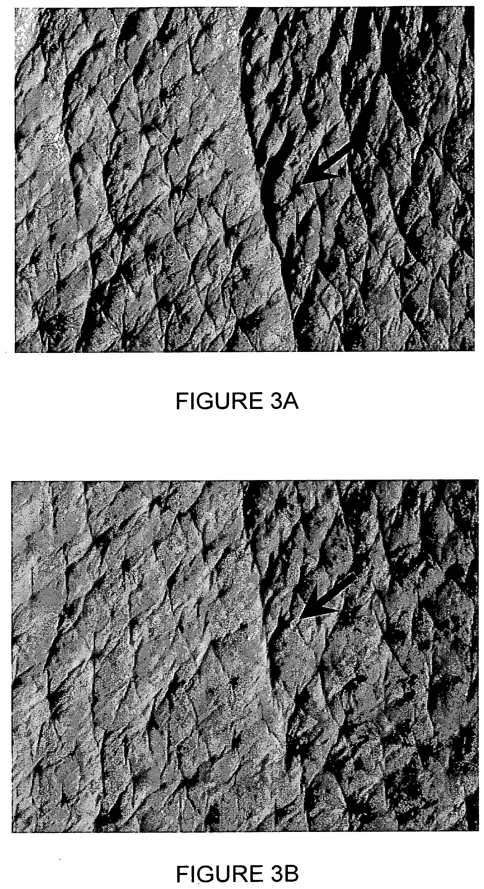

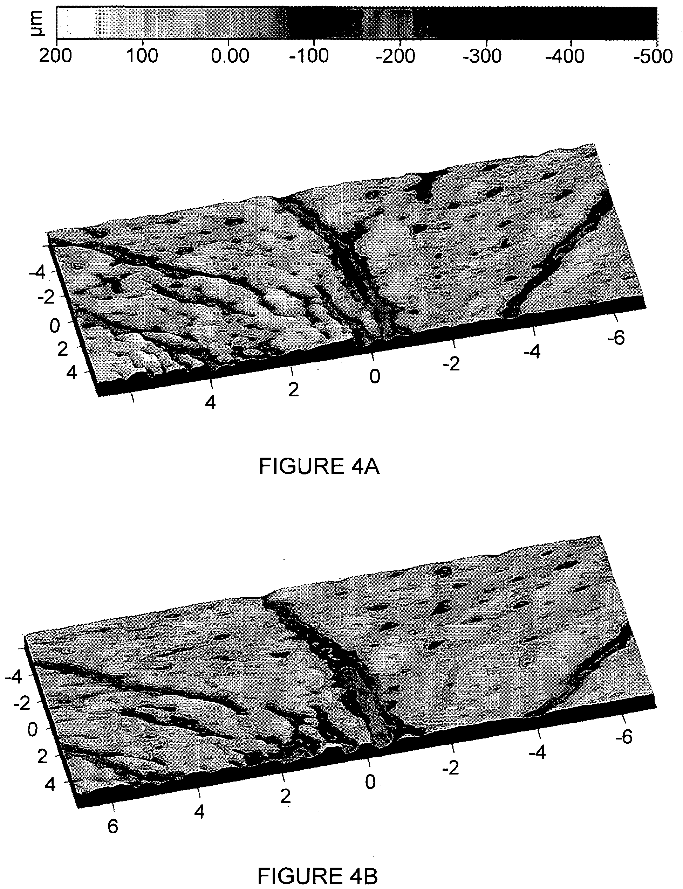

Figure 2 and Figure 3 represent examples of 2D visual measurements of skin area treated with product "T" (composition comprising 2% *Leucojum aestivum* extract, Figures 2A and 3A) or product "K" (control, Figures 2B and 3B) (Subject No. 8 and 4, respectively). Figure 4A represents 3D visual measurements obtained from skin areas of Subject No. 8 treated with product "T" (figure 4A) or product "K" (Figure 4B). Visible reduction in the wrinkle depth was observed.

**Example 4: Effect of *L. aestivum* bulb extract on MnSOD expression in keratinocytes**

*Leucojum aestivum* bulb extract, obtained as described hereinabove was previously demonstrated to stimulate the expression of the MnSOD gene. The aim of the present study was to examine whether the stimulation of gene expression is correlated with an increase in protein level. Protein expression was evaluated by flow cytometry.

Materials and methods

Biological model

Cellular type:    Normal human epidermal keratinocytes (NHEK) $K_{074}$ used at the 3rd passage
Culture conditions:    37°C, 5% $CO_2$
Culture medium:    Keratinocyte-SFM (Invitrogen 17005-034) supplemented with Epidermal Growth Factor (EGF) 0.25 ng/ml - Pituitary extract (PE) 25 μg/ml (Invitrogen 3700015) Gentamycine 25 μg/ml (Sigma G1397)
Assay medium:    Keratinocyte-SFM (Invitrogen 17005-034) supplemented with Gentamycine 25 μg/ml (Sigma G1397)

| Test compound | Form | Stock solution | Dilution | Test concentration |
|---|---|---|---|---|
| *Leucojum aestivum* **extract** | Liquid; Stored at +4°C | 5% in assay medium | Assay medium | 0.075, 0.15 and 0.3% |

Culture and treatment

The keratinocytes were cultivated in culture medium. At sub-confluence, the medium was removed and replaced by assay medium containing or not (control) the examined extract and the cells were incubated for 72 hours. All treatments were performed in n=3.

Flow cytometry analysis - Fluorescence-activated cell sorting (FACS)

At the end of the incubation, the cells were dissociated from their support by enzymatic treatment with trypsin.
After extensive washes, the cells were fixed in buffer solution and then labeled with an anti-MnSOD antibody (TEBU SOD-110C) prepared in a permeabilization buffer. After 1 hour of incubation and washes, the cells were incubated in the presence of a fluorescent conjugate against rabbit immunoglobulins (GAR-Alexa 488, Invitrogen A11008) prepared in a permeabilization buffer.
A control employing only the second antibody was also performed under the same conditions to verify the specificity of the immunolabelling.
After 1 hour of incubation and washes, the fluorescence parameters were measured by flow cytometry with a FACSArray™ cytometer driven by the FACSArray system software (Becton-Dickinson) on 10,000 individual cells (no cell population selection).

Data management

The raw data were analyzed with Microsoft Excel® software.
The inter-group comparisons were performed by Student's t-test. The statistical analysis can be interpreted if n≥5. For n<5, the statistical values can be considered informative but not quantitative. Formula used in this report is:

$$\text{Standard error of the mean:} \quad SEM = Sd/\sqrt{n}$$

The standard error of the mean (SEM) is a measure of how far the sample mean is likely to be from the true population mean. The SEM is calculated as the Standards Error (sd) divided by the square root of the sample size.

Results

As is shown in Table 9 hereinbelow, the *L. aestivum* extract used in the context of the invention, tested at 0.15%, slightly increased the MnSOD protein expression correlating with an increase of the gene expression. At concentrations of 0.075 and 0.3%, no effect of the compound on the MnSOD protein expression could be detected.

Table 9: Effect of Leucojum aestivum extract on MnSOD expression in keratinocytes

| Treatment | Concentration | Fluorescence Intensity (AU) | Mean (AU) | % of Control | SEM % | $P^{(1)}$ |
|---|---|---|---|---|---|---|
| Control | - | 1759 1921 1668 | 1782.7 | 100 | 4 | - |

(continued)

| Treatment | Concentration | Fluorescence Intensity (AU) | Mean (AU) | % of Control | SEM % | $P^{(1)}$ |
|---|---|---|---|---|---|---|
| *Leucojum aestivum* extract | 0.075% | 1917<br>1870<br>2067 | 1951.3 | 109 | 3 | ns |
| | 0.15% | 2185<br>2281<br>2013 | 2159.7 | 121 | 4 | * |
| | 0.3% | 2513<br>1780<br>2011 | 2101.3 | 118 | 12 | ns |
| $^{(1)}$: Threshold for statistical significance<br>ns : >0.05, Not significant<br>* : 0.01 to 0.05, Significant | | | | | | |

Conclusion

*Leucojum aestivum* bulb extract slightly increased the expression of the anti-oxidant enzyme MnSOD in keratinocytes. This effect was significant (p<0.05) at concentration of 0.15% extract and could be correlated to the increase of the gene expression by this extract.

**Example 5: Toxicity assessment of *L. aestivum* bulb extract**

Safety of *L. aestivum* bulb extract obtained as described hereinabove for cosmetic application was examined as follows:

Irritation

Irritation was examined by the "HET-CAM" test. Test principle is based on visual observation of possible irritations (hyperaemia, hemorrhaging, and coagulation/thrombosis) that may appear during the first five minutes following the application of the product on the chorioallantoic membrane of an embryonic chicken egg after ten days of incubation. The phenomena observed are registered according to their apparition (and not their intensity). (Operational mode ATS M1918LAC, which resumes the official method of the "Journal Officiel" according to the order of November 29th, 1996). The extract was found as non irritant, obtaining the score "0" in the HET-CAM test.

Cytotoxicity

Cytotoxicity was measured by the neutral red (NR) cytotoxicity procedure. This procedure is a cell survival/viability chemosensitivity assay, based on the ability of viable cells to incorporate and bind neutral red, a supra-vital dye. NR is a weak cationic dye that readily penetrates cell membranes by non-ionic diffusion, accumulating intracellularly in lysosomes, where it binds with anionic sites in the lysosomal matrix. Alterations of the cell surface or the sensitive lysosomal membrane lead to lysosomal fragility and other changes that gradually become irreversible. Such changes brought about by the action of xenobiotics result in a decreased uptake and binding of NR. It is thus possible to distinguish between viable, damaged, or dead cells. The test was performed with rabbit cornea fibroblasts that were contacted with the extract for a set period of time (Official Journal of the French Republic, December 27th 1999).

The extract was defined as having negligible cytotoxicity, as the percentage of cell mortality was lower than 20% at 50% dilution of the extract.

Cutaneous tolerance

Irritation was examined by the occlusive single patch test method basically as described in Dermatotoxicology Methods: The laboratory worker's VADEMECUM; N. Marzulli- H. Maibach Ed. Taylor & Francis, 1998. The extract was found as non-irritable after application to the skin, under patch, for 48 h.

**Claims**

1. Cosmetic method for slackening a cutaneous tissue comprising administering via subcutaneous, intradermal or transdermal delivery a composition comprising an aqueous extract of *Leucojum aestivum* bulbs in an amount effective in inhibiting muscle contraction, thereby slackening the cutaneous tissue and ameliorating, reducing, and/or eliminating at least one of skin wrinkles, skin fine lines and skin creases.

2. Cosmetic method according to claim 1, wherein the step of administering the composition includes one of subcutaneous injection, intradermal injection, or an application using iontophoresis.

3. Cosmetic method according to claim 2, wherein the injektion is a micro- or nano-injection or a needleless injection, or wherein the iontophoresis includes the use of liposomes, microspheres or transdermal patches.

4. Cosmetic method according to any of claims 1 to 3, wherein the extract is in a liquid form and the amount of the extract is from about 0.01% to about 99.9% (w/w) compared to the total weight of the composition.

5. Cosmetic method according to claim 4, wherein the amount of the extract is from about 0.01% to about 30% (w/w).

6. Cosmetic method according to any of claims 1 to 5, wherein the extract is in a dried form and the amount of the extract is from about 0.0001% to about 0.99% (w/w) compared to the total weight of the composition.

7. Cosmetic method according to claim 6, wherein the amount of the extract is from about 0.0001% to about 0.3% (w/w).

8. Cosmetic method according to any of claims 1 to 7, wherein the composition is a cosmetic composition further comprising a cosmetically effective diluent or carrier.

9. Cosmetic method according to claim 8, wherein the composition comprises at least one additive selected from the group consisting of fat, emulsifier and co- emulsifier, hydrophilic or lipophilic gelling agent, preservative, solvent, fragrance, filler, hydrophilic and lipophilic filter, dyestuffs, neutralizer, penetration enhancing agent and polymers.

10. Cosmetic method according to claim 8, wherein the composition comprises at least one additional active agent selected from the group consisting of de-pigmenting agent, moisturizer, anti-seborrheic agent, anti-acne agent, keratolytic and/or desquamating agent, anti-irritant agent, soothing agent and retinol.

**Patentansprüche**

1. Kosmetisches Verfahren zu Erschlaffung von Haut-Gewebe, welches umfasst, Verabreichen mittels subkutaner, intradermaler oder transdermaler Lieferung einer Zusammensetzung, welche einen wäßrigen Extrakt von Leucojum aestivum Knollen in einer Menge umfasst, die wirksam ist, Muskel-Kontraktionen zu inhibieren, wodurch das Haut-Gewebe gelockert und mindestens eines von Hautfältchen, feinen Hautlinien und Hautfalten verbessert, reduziert und/oder eliminiert wird.

2. Kosmetisches Verfahren nach Anspruch 1, worin der Schritt der Verabreichung der Zusammensetzung eines von subkutaner Injektion, intradermaler Injektion oder eine Anwendung unter Verwendung von Iontophorese umfasst.

3. Kosmetisches Verfahren nach Anspruch 2, worin die Injektion eine Mikro- oder Nano-Injektion oder eine Injektion ohne Nadeln ist oder worin die Iontophorese die Verwendung von Liposomen, Mikrokügelchen oder transdermalen Pflastern umfasst.

4. Kosmetisches Verfahren nach einem der Ansprüche 1 bis 3, worin der Extrakt in einer flüssigen Form vorliegt und die Menge des Extrakts von etwa 0,01 % bis etwa 99,9 % (Gew./Gew.) in Bezug zu dem Gesamtgewicht der Zusammensetzung ist.

5. Kosmetisches Verfahren nach Anspruch 4, worin die Menge des Extrakts von etwa 0,01 % bis etwa 30 % (Gew./Gew.) ist.

6. Kosmetisches Verfahren nach einem der Ansprüche 1 bis 5, worin der Extrakt in einer getrockneten Form vorliegt und worin die Menge des Extrakts von etwa 0,0001 % bis etwa 0,99 % (Gew./Gew.) ist, bezogen auf das Gesamtgewicht der Zusammensetzung.

7. Kosmetisches Verfahren nach Anspruch 6, worin die Menge des Extrakts von etwa 0,0001 % bis etwa 0,3 % (Gew./Gew.) ist.

8. Kosmetisches Verfahren nach einem der Ansprüche 1 bis 7, worin die Zusammensetzung eine kosmetische Zusammensetzung ist, die weiter ein kosmetisch wirksames Verdünnungsmittel oder einen kosmetisch wirksamen Träger enthält.

9. Kosmetisches Verfahren nach Anspruch 8, worin die Zusammensetzung mindestens ein Additiv enthält, ausgewählt aus der Gruppe bestehend aus Fett, Emulgator, und Co-Emulgator, hydrophilen oder lipophilen Gelierungsmitteln, Konservierungsmitteln, Lösungsmitteln, Duftstoffen, Füllmitteln, hydrophilen oder lipophilen Filtern, Farbstoffen, Neutralisierungsmitteln, Eindring-Verstärkungsmitteln und Polymeren.

10. Kosmetisches Verfahren nach Anspruch 8, worin die Zusammensetzung mindestens ein weiteres aktives Mittel enthält, ausgewählt aus der Gruppe bestehend aus Entfärbungsmitteln, Befeuchtern, anti-seborrhohische Mitteln, anti-Akne-Mitteln, keratolytischen und/oder anti-Schuppen-Mitteln, anti-Reizmitteln, Beruhigungsmitteln und Retinol.

**Revendications**

1. Procédé cosmétique pour relâcher un tissu cutané comprenant l'administration par distribution sous-cutanée, intradermique ou transdermique d'une composition comprenant un extrait aqueux de bulbes de Leucojum aestivum en une quantité efficace pour inhiber la contraction musculaire, ce qui permet de relâcher le tissu cutané et d'améliorer, de réduire et/ou d'éliminer au moins l'un(e) parmi les rides, les lignes fines et les plis de la peau.

2. Procédé cosmétique selon la revendication 1, l'étape d'administration de la la composition comprenant l'une parmi une injection sous-cutanée, une injection intradermique, ou une application par iontophorèse.

3. Procédé cosmétique selon la revendication 2, l'injection étant une micro- ou nano-injection ou une injection sans aiguille, ou l'iontophorèse comprenant l'utilisation de liposomes, de microsphères ou de patchs transdermiques.

4. Procédé cosmétique selon l'une quelconque des revendications 1 à 3, l'extrait étant sous forme liquide et la quantité de l'extrait étant d'environ 0,01% à environ 99,9% en poids par rapport au poids total de la composition.

5. Procédé cosmétique selon la revendication 4, la quantité de l'extrait étant d'environ 0,01% à environ 30% en poids.

6. Procédé cosmétique selon l'une quelconque des revendications 1 à 5, l'extrait étant sous forme sèche et la quantité de l'extrait étant d'environ 0,0001% à environ 0,99% en poids par rapport au poids total de la composition.

7. Procédé cosmétique selon la revendication 6, la quantité de l'extrait étant d'environ 0,0001% à environ 0,3% en poids.

8. Procédé cosmétique selon l'une quelconque des revendications 1 à 7, la composition étant une composition cosmétique comprenant en outre un diluant ou un support cosmétiquement efficace.

9. Procédé cosmétique selon la revendication 8, la composition comprenant au moins un additif sélectionné dans le groupe constitué par une graisse, un émulsifiant et un co-émulsifiant, un agent gélifiant hydrophile ou lipophile, un conservateur, un solvant, un parfum, une charge, un filtre hydrophile et lipophile, des colorants, un neutralisant, un agent favorisant la pénétration et des polymères.

10. Procédé cosmétique selon la revendication 8, la composition comprenant au moins un agent actif supplémentaire sélectionné dans le groupe constitué par un agent de dépigmentation, un agent hydratant, un anti-séborrhéique, un agent antiacné, un agent kératolytique et/ou desquamant, un anti-irritant, un calmant et le rétinol.

140
120
100
80
60
40
20
0

T0
2h
6h
24h

||||||| Negative control

▓▓▓ Positive control

░░░ 1.5% *L. aestivum* extract

FIGURE 1

FIGURE 2A

FIGURE 2B

FIGURE 3A

FIGURE 3B

FIGURE 4A

FIGURE 4B

FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6395277 B
- US 6939852 B
- US 7384918 B
- US 20080069912 A
- US 6573376 B
- US 6617452 B
- US 20090216012 A
- US 6117428 A
- US 6159476 A
- US 7029708 B
- US 5958903 A
- US 5965571 A
- US 20060160702 A
- US 20080254055 A
- BG 1055 U1
- WO 9836761 A
- US 4708861 A
- US 4224179 A
- US 4235871 A

### Non-patent literature cited in the description

- Remington's Pharmaceutical Sciences. Mack Publishing Co,
- Liposome Technology. CRC Press, 1991, vol. I, II, III
- **DAVIS S. S. ; WALKER I. M.** *Methods in Enzymology,* 1987, vol. 149, 51-64
- **MAYHEW E. et al.** *Methods in Enzymology,* 1987, vol. 149, 64-77
- **SHAFER-KORTING M. et al.** *J. Am. Acad. Dermatol.,* 1989, vol. 21, 1271-1275
- *Journal Officiel,* 29 November 1996
- *Official Journal of the French Republic,* 27 December 1999
- Dermatotoxicology Methods: The laboratory worker's. Taylor & Francis, 1998